# EUROPÄISCHE PATENTSCHRIFT

(11) **EP 0 997 132 B1**
(45) Veröffentlichungstag und Bekanntmachung des Hinweises auf die Patenterteilung: **28.02.2007**
(21) Anmeldenummer: 99118560.4
(22) Anmeldetag: 20.09.1999
(51) Int. Cl.: A61K 6/083, C03C 3/066, C03C 3/118

(54) **Bariumfreies röntgenopakes Dentalglas und Dentalglas-Kunststoff-Komposit**
Barium-free radiopaque dental glass and polymer dental glass composite material
Verre dentaire radiopaque exempt de barium, composite de verre dentaire et de polymère

(30) Priorität: 27.10.1998 DE 19849388
(43) Veröffentlichungstag der Anmeldung: 03.05.2000
(73) Patentinhaber: Schott AG, 55122 Mainz (DE)
(72) Erfinder: Kunert, Christian, 55118 Mainz (DE); Kessler, Susanne, 84030 Ergolding (DE); Paschke, Hartmut, Dr., 84030 Ergolding (DE); Weitzel, Alwin, 55120 Mainz (DE); Wölfel, Ute, 55130 Mainz (DE)

(56) Entgegenhaltungen:
- EP-A- 0 241 277
- EP-A- 0 716 049
- DE-C- 4 323 143
- GB-A- 2 202 221
- US-A- 4 358 549
- US-A- 5 215 459

## Beschreibung

Die Erfindung betrifft ein bariumfreies röntgenopakes Dentalglas, ein das Glas enthaltendes Dentalglas-Kunststoff-Komposit.

Für Zahnfüllungen werden in zunehmendem Maße Dentalglas-Kunststoff-Komposite eingesetzt, um mögliche Nebenwirkungen von Amalgam-Füllungen zu umgehen und um einen besseren ästhetischen Eindruck zu erzielen. Dentalglas-Kunststoff-Komposite bestehen in der Regel aus einem anorganischen Anteil und einem organischen Kunststoff-Binder. Der anorganische Anteil besteht überwiegend aus Glaspulver. An das verwendete Glaspulver werden neben den für eine gute Füllung notwendigen Pulvereigenschaften auch noch bestimmte Anforderungen an die physikalischen und chemischen Eigenschaften des für das Pulver zu verwendenden Glases gestellt.

Das Glaspulver muß hinsichtlich der Brechzahl möglichst gut an die verwendete Kunstharzmatrix angepaßt sein, um die teiltransparente Erscheinung von natürlichem Zahnschmelz zu imitieren und damit den hohen ästhetischen Anforderungen zu genügen.

Brechzahldifferenzen zwischen Glas und Kunstharz über 0,05 bewirken eine unerwünscht hohe Opazität des Dentalglas-Kunststoffverbundes und sind daher zu vermeiden.
Darüber hinaus muß das Glaspulver bei der Herstellung der Komposite eine gute Verarbeitbarkeit sowie ein günstiges Abbindeverhalten besitzen und nach der Aushärtung eine hohe Festigkeit gewährleisten.

Weiterhin wichtig ist, daß die thermische Ausdehnung des Dentalglas-Kunststoff-Komposits im Verwendungsbereich der Füllung, d. h. bei Temperaturen zwischen 30°C und 70°C der des Zahnmaterials angepaßt ist, um sicherzustellen, daß die Füllung eine ausreichende Temperaturwechselbeständigkeit aufweist. Gerade durch den Wechsel von kalten und heißen Speisen ist hier die Gefahr gegeben, daß sich die Füllung durch derartige thermische Wechselbelastung lockert und sich somit ein Spalt zwischen Füllung und Zahn bildet, der einen bevorzugten Angriffspunkt für Sekundärkaries darstellt.

Üblich ist ein möglichst kleiner Ausdehnungskoeffizient für das Glas, weil damit die verhältnismäßig hohe thermische Ausdehnung des Kunstharz-Binders kompensiert werden kann.

Die Röntgenopazität von Dentalgläsern oder -materialien wird nach DIN ISO 4049 relativ zur Röntgenabsorption von Aluminium als AluminiumGleichwertdicke (Al-GWD) angegeben. Die Al-GWD ist die Dicke einer Aluminium-Probe, die die gleiche Absorption bewirkt wie eine 2 mm dicke Probe des zu prüfenden Materials. Eine Al-GWD von 4 mm bedeutet also, daß ein Glasplättchen von 2 mm Dicke dieselbe Röntgenschwächung bewirkt wie ein Aluminiumplättchen von 4 mm Dicke. Von röntgenopaken Dentalgläsern wird eine Al-GWD von mindestens 4 mm gefordert. Dadurch ist im Einsatz als Zahnfüllung auf Röntgenaufnahmen eine ausreichend gute Unterscheidbarkeit zwischen Füllung und Zahnsubstanz sichergestellt. Auftretende Spalten und Karies können gut erkannt werden.

Weiterhin muß eine gute chemische Beständigkeit des Glaspulvers gegen Wasser, Säuren und Laugen zu einer langen Lebensdauer der Zahnfüllung beitragen. Wegen möglicher toxischer Nebenwirkungen soll auf die Verwendung von Barium-Bestandteilen in dem Glas verzichtet werden, obwohl diese Bestandteile eine gute Röntgenopazität hervorrufen. Die Verwendung von bleihaltigen Bestandteilen ist aus toxischen Gesichtspunkten ebenfalls unerwünscht.

Die DE 32 48 357 A1 beschreibt einen pulverförmigen Dentalwerkstoff auf der Grundlage von Calciumaluminiumfluorosilicatgläsern (a) und für Dentalzwecke üblichen Metallen (b) und weiteren Komponenten, der dadurch gekennzeichnet ist, daß er wenigstens einen Teil von (a) als gesinterte Mischung mit (b) enthält. Die verwendeten Pulver von (a) bestehen aus (Gew.-% berechnet als Oxide) SiO₂ 20 - 60, Al₂O₃ 10- 50, CaO 1 - 40, F 1 - 40, Na₂O 0 - 10, P₂O₅ 0 - 10 und insgesamt 0 - 20 Gew.-%, berechnet als Oxide, an B, Bi, Zn, Mg, Sn, Ti, Zr, La oder anderen dreiwertigen Lanthanidoxiden, K, W, Ge.

Die US 5,215,459 betrifft die Verwendung von Glasionomerzementen für gesteuerte Geweberegenerationen. Die für das Glaspulver genannten Zusammensetzungsbereiche entsprechen den in DE 32 48 357 A1 genannten mit zusätzlich SrO als fakultativen Bestandteil mit 0 - 40 Gew.-%, wobei CaO und/oder SrO wenigstens 1 Gew.-% beträgt. Um das Glas röntgensichtbar zu machen, können 10 bis 20 Gew.-% La₂O₃ hinzugefügt werden.

Die in den oben genannten Schriften beschriebenen Gläser weisen einen verhältnismäßig geringen Gesamtgehalt an B₂O₃, ZnO, ZrO₂ und La₂O₃ (≤ 20 Gew.-%) auf.

Die US 4,775,592 beschreibt ein Fluoroaluminosilicatglas-Pulver für die Verwendung als Dentalglasionomerzement, dessen Oberfläche mit einem Metallfluorid oder einem Fluoro-Komplexsalz nachbehandelt ist. Die aufwendige Nachbehandlung dient hierbei dazu, die erforderlichen Verarbeitungseigenschaften sowie eine hohe Druckfestigkeit des Zements zu erzielen. Die Zusammensetzung des verwendeten Fluoroaluminosilicatglas-Pulvers kann in einem weiten Zusammensetzungsbereich liegen. Es wird hergestellt durch Erschmelzen der Komponenten in (Gew.-%) SiO₂ 25 - 50, Al₂O₃ 15 - 40, F 10 - 40 und Phosphat 0 - 20. Dabei kann F als Fluorid von Zn, Al, Y, La, Zr, Alkalien und Erdalkalien und Phosphat als Phosphat von Alkalien, Erdalkalien, Zn, Al, Y, La, Zr eingeführt werden. In das Glas können auch Y-, La-, Zn-, Ti-, Zr- und Erdalkalioxide eingeführt werden.

In der JP 61-215234 A wird eine Glaszusammensetzung für die Verwendung als Glasionomerzement, geeignet als Dentalzement, beansprucht. Es wird ein weitgefaßter Zusammensetzungsbereich, bestehend aus einer Vielzahl möglicher Komponenten beansprucht. Nach der Schrift ist es dennoch nur möglich, Gläser mit einer relativ niedrigen Brechzahl in einem zudem engen Bereich von 1,46 bis 1,60 einzustellen. Gläser mit Brechzahlen im für neuartige Dentalmaterialien günstigen Bereich > 1,60 werden nicht beschrieben. Dabei ist ein Ba-Gehalt von bis zu 35 Gew.-% möglich. Der in zwei Beispielen angegebene Ba-Gehalt von 20,31 und 3,92 Gew.-% ist aus toxikologischen Gründen bedenklich und entspricht nicht den Erfordernissen an moderne Dentalgläser.

Auffallend bei dieser Schrift und der US 4,775,592 ist der beanspruchte hohe F-Gehalt von 10 bis 40 Gew.-% sowie der fakultative Gehalt an B³⁺ und P⁵⁺ von jeweils 0 bis 8 Gew.-% (JP 61-215234 A) bzw. Phosphat 0 bis 20 Gew.-% (US 4,775,592). Die Herstellung eines ungetrübten Dentalglases mit hohem F-Gehalt ohne zwingende Anwesenheit von B³⁺ und/oder P⁵⁺ ist schwierig.

Die US 3,971,754 beschreibt die Herstellung eines Zahnfüllmaterials unter Verwendung eines barium-, zink- und zirkoniumfreien Glases, welches zur Einstellung einer Röntgenopazität Oxide und Carbonate von Lanthan, Hafnium, Strontium oder Tantal im Bereich von 5 bis 60 Gew.-% enthält.

Die JP 6-39031 A beschreibt zinkfreie röntgenopake Implantatmaterialien auf der Basis von Calcium- und Strontiumapatit-Glaskeramiken. In den beschriebenen Zusammensetzungen wird als röntgenabsorbierende Komponente in nahezu allen Fällen ausschließlich SrO eingesetzt, lediglich in 2 Beispielen wird bis zu 5 Gew.-% ZrO₂ verwendet. Flußmittel wie Na₂O oder B₂O₃ sind allenfalls in sehr geringen Mengen (max. 0,5 Gew.-%) enthalten.

Die JP 5-331017 A beschreibt zink- und zirkoniumfreie Glaspulver für Dentalzemente, deren röntgenabsorbierende Wirkung auf dem Einsatz von SrO und La₂O₃ (bis 20 Gew.-%) beruht.

In der DE 3788816 T2 wird ein Verfahren zur Herstellung von radioopakem, vernetztem Poly(carbonsäure)-Zahnzement mit einem fluorhaltigen, zink- und zirkoniumfreien Glaspulver beschrieben. Die notwendige Röntgenabsorption wird durch einen Zusatz von 5 bis 35 Gew.-% SrO eingestellt.

In der US 4,215,033 wird ein Dental-Harz-Komposit beansprucht, bestehend aus einem Harz und einem nichttoxischen, alkali- und fluoridfreien Füllstoff, wobei der Füllstoff aus einem zweiphasigen Boroaluminosilicat-Glas besteht und eine Phase teilweise wieder entfernt wird. Das Glas kann Zusätze von SrO, CaO und ZnO oder SrO/ZrO₂ enthalten.

Die DE 44 43 173 C2 beansprucht ebenfalls ein bariumfreies, hoch siliciumhaltiges (50 bis 75 Gew.-% SiO₂) Dentalglas mit guter Röntgenabsorption.

Weiterhin ist aus der DE 43 23 143 C1 ein barium-, zink- und zirkoniumfreies Dentalglas mit hoher Röntgenabsorption und einer Brechzahl n_{d} ≤ 1,56 bekannt, das eine Zusammensetzung in Gew.-% auf Oxidbasis besitzt von SiO₂ 45-65, B₂O₃ 5-20, Al₂O₃ 5-20, CaO 0-10, SrO 15-35 und F₂-O 0-2. Die gute Röntgenopazität wird hier durch einen verhältnismäßig hohen Anteil an SrO erreicht.

Aufgabe der Erfindung ist es, ein bariumfreies röntgenopakes Dentalglas für die Verwendung in Dentalglas-Kunststoff-Kompositen zu finden sowie ein Dentalglas-Kunststoff-Komposit, das ein solches Dentalglas enthält, bereitzustellen. Das Dentalglas und das Dentalglas-Kunststoff-Komposit sollen dabei preiswert und dennoch hochwertig und körperverträglich sowie zum passiven und aktiven Zahnschutz geeignet sein und hinsichtlich der Verarbeitbarkeit, des Abbindeverhaltens und der Festigkeit vorzügliche Eigenschaften aufweisen.
Die Brechzahl n_{d} des Dentalglases soll an die zur Verfügung stehenden Dentalkunststoffe, besonders an solche mit einer Brechzahl n_{d} > 1,60, angepaßt sein, und so den an ein Dentalglas-Kunststoff-Komposit gestellten ästhetischen Anforderungen nach einem natürlichen Aussehen genügen

Diese Aufgabe wird durch das in Anspruch 1 beschriebene Dentalglas und durch das in Anspruch 11 beschriebene Dentalglas-Kunststoff-Komposit gelöst.

Das erfindungsgemäße Dentalglas erreicht die Eigenschaften bariumhaltiger Dentalgläser bezüglich der geforderten Röntgenabsorption ohne Einsatz von Bariumverbindungen oder anderer gesundheitlich bedenklicher Substanzen.

Im Gegensatz zu herkömmlichen Dentalgläsern wird die benötigte Röntgenopazität nicht nur durch eine Komponente allein bewirkt, sondern vielmehr durch eine Kombination verschiedener röntgenabsorbierender Elemente, deren Wirkungen sich vorzugsweise gegenseitig ergänzen, da sie unterschiedliche Bereiche der Strahlung der Röntgenröhre absorbieren.

Der Mindestgehalt an ZnO in den erfindungsgemäßen Gläsern ermöglicht die Ausnutzung der bakteriostatischen Wirkung von Zn²⁺, insbesondere im kritischen Grenzbereich zwischen Füllung und umgebenden Zahn.
Die Brechzahl n_{d} des Dentalglases läßt sich über einen weiten Bereich von 1,47 bis 1,70 variieren, wobei über den gesamten Bereich die genannten weiteren Anforderungen erfüllt werden.

Die Brechzahlen n_{d} der Dentalgläser entsprechen denen der üblichen zur Verfügung stehenden Dentalkunststoffe. Für einen bestimmten Dentalkunststoff mit vorgegebener Brechzahl n_{d}, insbesondere auch für neuere hochbrechende Kunstharze mit n_{d} > 1,6, wie beispielsweise in US 5,679,710 beschrieben, wird ein Dentalglas mit übereinstimmender Brechzahl bereitgestellt. Damit läßt sich ein dem natürlichen Zahnschmelz entsprechendes Erscheinungsbild des Dentalglas-Kunststoff-Komposits ermöglichen.

Das Glas enthält 20-45 Gew.-% SiO₂ als glasbildenden Bestandteil. Bei niedrigeren Gehalten steigt die Kristallisationsneigung in unzulässiger Weise an, so daß keine für den gewünschten Einsatzzweck geeigneten klaren Gläser erhalten werden können. SiO₂-Gehalte über 45 Gew.-% führen zu unvorteilhaft hohen Schmelztemperaturen, während gleichzeitig die hohe Röntgenopazität und der Mindestfluoridgehalt nicht erreicht werden können.
Al₂O₃ wird im Bereich 5-35 Gew.-% und P₂O₅ im Bereich 0-10 Gew.-% eingesetzt. Der Mindestgehalt an Al₂O₃ ist erforderlich, um ein Netzwerk mit geeigneten strukturellen Einheiten zu schaffen, die einen Einbau von Fluorid im erforderlichen Maß und somit die Herstellung klarer Gläser ermöglichen. Insbesondere bei hohen Fluoridgehalten wird dazu auch vorzugsweise P₂O₅ eingesetzt. Höhere Al₂O₃-Gehalte als 35 Gew.-% führen zu unvorteilhaft hohen Schmelztemperaturen, P₂O₅-Gehalte über 10 Gew.-% bewirken eine hohe Entmischungsneigung der Gläser sowie eine für die weitere Verarbeitung der Gläser und den späteren Einsatz unzureichende chemische Beständigkeit. 1-10 Gew.-% Na₂O werden in den erfindungsgemäßen Gläsern als Flußmittel zur Senkung der Schmelztemperatur eingesetzt. Denselben Zweck erfüllen K₂O und Cs₂O, welche gegebenenfalls zusätzlich eingesetzt werden können, wobei der Gesamtalkaligehalt der Gläser 15 Gew.-% nicht überschreiten sollte, um eine ausreichende chemische und mechanische Beständigkeit zu gewährleisten. Der Einsatz von K₂O und Cs₂O ist insbesondere dann empfehlenswert, wenn zur Erzielung einer besonders hohen Röntgenopazität bei gleichzeitig hoher Brechzahl die Forderung nach preisgünstigen Rohstoffen an Bedeutung verliert. Der Gehalt soll jedoch auf je maximal 8 Gew.-% beschränkt sein.
B₂O₃ kann ähnlich den Alkalien in Gehalten bis 10 Gew.-% als Flußmittel eingesetzt werden. Neben der erniedrigenden Wirkung auf die Schmelztemperatur führt der Einsatz von B₂O₃ gleichzeitig zu einer Verbesserung der Kristallisationsstabilität der Gläser, so daß auch bei höheren Fluoridgehalten noch klare, nicht kristallisierende Gläser erhalten werden können. Höhere Konzentrationen als 10 Gew.-% sind nicht zu empfehlen, da sonst die chemische Beständigkeit zurückgeht.
ZnO wird im Bereich zwischen 2 und 20 Gew.-% eingesetzt. Bei Gehalten unter 2 Gew.-% ist die geforderte bakteriostatische Wirkung der mit den erfindungsgemäßen Gläsern hergestellten Dentalmaterialien nicht mehr sichergestellt. Höhere Gehalte als 20 Gew.-% führen zu einer verschlechterten chemischen Beständigkeit. Zudem wird dann die Grenze der Löslichkeit für ZnO für dieses Glassystem erreicht, so daß Kristallisationsprobleme auftreten. Des weiteren wirkt sich die Zugabe von ZnO günstig auf das Abbindeverhalten aus.
Zusammen mit dem genannten ZnO-Gehalt gewährleistet ein ZrO₂-Gehalt zwischen 2 und 10 Gew.-% eine ausreichende Röntgenabsorption der erfindungsgemäßen Gläser. Ein Mindestgehalt von 2 Gew.-% garantiert zudem die gewünschte chemische Beständigkeit; die mechanischen Eigenschaften, und besonders die Zug- und Druckfestigkeit werden hierbei verbessert, während sich mit ZrO₂-Gehalten über 10 Gew.-% der Brechwert n_{d} nicht im gewünschten Bereich zwischen 1,47 und 1,70 einstellen läßt und gleichzeitig die Schmelztemperaturen und insbesondere die Kristallisationsneigung in unerwünschter Weise ansteigen.
Insbesondere bei niedrigen ZnO- und ZrO₂-Gehalten ist für eine hohe Röntgenopazität die Zugabe von bis zu 27 Gew.-% SrO empfehlenswert. Die Zugabe von SrO beeinflußt die Brechzahl und wirkt sich günstig auf die Schmelzeigenschaften und das Abbindeverhalten aus. SrO-Gehalte über 27 Gew.-% führen jedoch zu verstärkter Kristallisation und sollten vermieden werden. Insbesondere bei SrO-freien Gläsern ist es bevorzugt, durch eine Zugabe von bis zu 8 Gew.-% CaO das gewünschte Abbindeverhalten zu fördern. Durch Zugabe von bis zu 10 Gew.-% La₂O₃ läßt sich die geforderte hohe Röntgenabsorption besonders gut einstellen. Die charakteristische Röntgenabsorption von ZnO, ZrO₂ und SrO wird insbesondere durch die charakteristische Röntgenabsorption von La₂O₃ hervorragend ergänzt. Dadurch erhält man eine ausreichend hohe Röntgenabsorption über den gesamten Energiebereich der für medizinische Zwecke verwendeten Röntgenstrahlung.
Um die an Dentalgläser gestellten Anforderungen zu erfüllen, muß der Gesamtgehalt der Komponenten B₂O₃, ZnO, ZrO₂ und La₂O₃ mindestens größer 20 Gew.-% betragen.
Fluorid, eingesetzt als Kryolith (Na₃AlF₆), AlF₃, SrF₂ oder als Fluorid der weiteren eingesetzten Elemente, dient im Bereich zwischen 2 und 20 Gew.-% neben der Erzielung einer niedrigen Brechzahl n_{d} auch als erwünschtes Fluoriddepot im Dentalmaterial, welches im Laufe der Zeit Fluorid an die umgebende Zahnsubstanz abgibt. Außerdem wird die Herstellung klarer Gläser ermöglicht. Dafür ist ein Mindestgehalt von 2 Gew.-% erforderlich. Gehalte über 20 Gew.-% sind zu vermeiden, da dann die Entmischungs- und Kristallisationsneigung der Gläser bei der Herstellung drastisch ansteigt. Zudem ist dann mit erheblichen Fluoridverlusten bei der Schmelze zu rechnen, was einen deutlich erhöhten Aufwand beim Personenschutz und bei der Vermeidung umweltgefährdender Dämpfe erfordert. Der Einsatz von Kryolith als Fluoridrohstoff berücksichtigt die Anforderung, für die Herstellung möglichst preisgünstige Rohstoffe einzusetzen.

Die Brechzahl des erfindungsgemäßen Dentalglases läßt sich dabei im Bereich von 1,47 bis 1,70 einstellen. Damit das Erscheinungsbild der Dentalglas-Kunststoff-Komposite dem von natürlichen Zahnschmelz nahekommt, wird die Brechzahl von Dentalglas und Dentalkunststoff angepaßt. Erfindungsgemäße Dentalgläser mit einer Brechzahl > 1,60 sind insbesondere für den Einsatz von zukunftsträchtigen hochbrechenden Kunstharzen, wie in US 5,679,710 beschrieben, geeignet.

Brechzahlen im Bereich von 1,47 bis 1,59 lassen sich durch ein Dentalglas der Zusammensetzung (in Gew.-%) SiO₂ 20 - 45, Al₂O₃ 7 - 35, B₂O₃ 0,5 - 10, Na₂O 2 - 10,K₂O 0 - 8, Cs₂O 0 - 8, Summe der Alkalimetall-Oxide 2 - 10, CaO 0-5, SrO 0 - 25, ZnO 2- 15, ZrO₂ 2 - 6, P₂O₅ 2- 10, La₂O₃ 0-5 und F 7 - 20 einstellen. Der Gesamtgehalt an B₂O₃, ZnO, ZrO₂ und La₂O₃ ist > 20 Gew.-%.

Brechzahlen im Bereich von 1,49 bis 1,57 lassen sich durch ein Dentalglas der Zusammensetzung (in Gew.-%) SiO₂ 20 - 44, Al₂O₃ 12 - 22, B₂O₃ 5 - 10, Na₂O 2 - 8, CaO 0 - 4, SrO 0-18,5, ZnO 3 -15, ZrO₂ 3 - 6, P₂O₅ 4-10, La₂O₃ 0 - 4 und F 10 - 20 einstellen. Der Gesamtgehalt an B₂O₃, ZnO, ZrO₂ und La₂O₃ ist > 20 Gew.-%.

Brechzahlen im Bereich von 1,59 bis 1,70 lassen sich durch ein Dentalglas der Zusammensetzung (in Gew.-%) SiO₂ 20 - 30, Al₂O₃ 5 - 25, B₂O₃ 0 -10, Na₂O 3 - 10, K₂O 0 - 8, Cs₂O 0 - 8, Summe der Alkalimetall-Oxide 3 - 15, CaO 0 -8, SrO 0 - 25, ZnO 2 -20, ZrO₂ 2 - 10, P₂O₅ 0 - 10, La₂O₃ 0 - 10 und F 2 - 10 einstellen. Der Gesamtgehalt an B₂O₃, ZnO, ZrO₂ und La₂O₃ ist > 20 Gew.-%. Diese Dentalgläser zeichnen sich durch eine besonders gute Röntgenopazität aus.

Insbesondere für hochbrechende Dentalkunststoffe mit einer Brechzahl n_{d} > 1,60 stehen mit den erfindungsgemäßen Dentalgläsern erstmalig bariumfreie röntgenopake Füllgläser mit angepaßter Brechzahl zur Verfügung.

Brechzahlen im Bereich von 1,59 bis 1,67 lassen sich durch ein Dentalglas der Zusammensetzung (in Gew.-%) SiO₂ 20 - 30, Al₂O₃ 5 - 25, B₂O₃ 1- 10, Na₂O 3 - 10, K₂O 0 - 8, Cs₂O 0 - 8, Summe der Alkalimetall-Oxide 5 - 15, CaO 0 - 5, SrO 10 - 25, ZnO 8 - 20, ZrO₂ 4-10, P₂O₅ 2-10, La₂O₃ 3-10 und F 2 - 7 einstellen. Der Gesamtgehalt an B₂O₃, ZnO, ZrO₂ und La₂O₃ ist > 20 Gew.-%.

Brechzahlen im Bereich von 1,59 bis 1,66 lassen sich durch ein Dentalglas der Zusammensetzung (in Gew.-%) SiO₂ 20 - 30, Al₂O₃ 5 - 15, B₂O₃ 2- 5, Na₂O 3 - 7, K₂O 0 - 5, Cs₂O 0 - 5, Summe der Alkalimetall-Oxide 5 - 13, CaO 0 - 5, SrO 15 - 24, ZnO 10 - 15, ZrO₂ 4 -9, P₂O₅ 2 - 5, La₂O₃ 3 - 8 und F 2 - 5 einstellen. Der Gesamtgehalt an B₂O₃, ZnO, ZrO₂ und La₂O₃ ist > 20 Gew.-%.

Die erfindungsgemäßen Gläser können neben SrO, ZrO₂, ZnO und La₂O₃ als weitere röntgenabsorbierende Komponenten Oxide der Gruppen (Sc₂O₃, Y₂O₃, Nb₂O₅, Gd₂O₃, Yb₂O₃) und (HfO₂, Ta₂O₅, WO₃) bis zu je 10 Gew.-% pro Gruppe enthalten. Für die Herstellung von preisgünstigen Gläsern mit niedrigen Brechzahlen werden diese Komponenten vorzugsweise nicht eingesetzt. In diesen Fällen wird die Röntgenopazität durch eine Kombination aus ZnO, ZrO₂, SrO und ggf. La₂O₃ gewährleistet.

Für die zahnärztliche Praxis ist die gute Erkennbarkeit der Füllung im Röntgenbild von hoher Bedeutung. Die erfindungsgemäßen Dentalgläser besitzen Aluminiumgleichwertdicken von mindestens 4 mm und erfüllen damit die erforderlichen Eigenschaften für die Verwendung in der Zahnrestauration.

Das Dentalglas kann aufgrund seiner Zusammensetzung, aus bei der Glasherstellung gängigen Verbindungen, preiswert hergestellt werden. Durch die vorteilhafte Kombination dieser Verbindungen konnte ein, bezüglich Festigkeit, Abbindeverhalten und Verarbeitbarkeit, hochwertiges und körperverträgliches Dentalglas gefunden werden.
Ein erfindungsgemäßes Dentalglas-Kunststoff-Komposit und ein hierfür geeignetes Dentalglas zeichnet sich nicht nur durch die hervorragende Verwendung zum passiven Zahnschutz aus, z. B. in Form von Zahnfüllungen, sondern kann aufgrund seiner Zusammensetzung, besonders durch die Verwendung der bakteriostatischen Komponente Zink und Fluorid, aktiv am präventiven Zahnschutz mitwirken.

Die erfindungsgemäßen Gläser werden folgendermaßen hergestellt:
Die Rohstoffe, bevorzugt Carbonate und Fluoride, werden abgewogen und anschließend gründlich gemischt. Das Glasgemenge wird bei ca. 1400 - 1540°C eingeschmolzen und gut homogenisiert. Die Temperatur beim Gießen beträgt 1280-1460°C. Der Guß erfolgt vorzugsweise auf wassergekühlte Stahlplatten oder -walzen. Die klaren Glasplättchen mit Dicken bis 2 mm können anschließend leicht mit bekannten Mitteln zu Glaspulvern für Dentalanwendungen aufgemahlen werden. Ein Schmelzbeispiel zur Herstellung eines Dentalglases entsprechend Beispiel 1.4 ist in Tabelle 3 angeführt.

Nach seiner Herstellung wird aus dem Glas in an sich bekannter Weise z. B. durch Mahlen und ggf. Sieben ein Glaspulver hergestellt, das die für Dentalzwecke übliche mittlere Teilchengröße von ≤ 10 µm, insbesondere 0,5 bis 5 µm, bevorzugt 0,7 bis 1,5 µm besitzt. Die Pulverkörnung spielt eine wichtige Rolle, sie beeinflußt die Polierbarkeit der Komposite, sowie die Abrasions- und mechanische Festigkeit. Zur Erzielung guter mechanischer Eigenschaften ist in üblicher Weise eine nicht zu enge Korngrößenverteilung günstig, wie sie z. B. durch übliche Vermahlung und Absiebung der Grobanteile erreicht wird. Eine maximale Teilchengröße von 40 µm, vorzugsweise 20 µm, insbesondere 10 µm sollte nicht überschritten werden. In dieser Form ist das Glaspulver zur Verwendung als Füllmittel für als Zahnfüllungen verwendete Dental-Komposite besonders geeignet.

Es ist vielfach üblich, die Dentalglaspulver zu silanisieren, wobei die Silanisierung sowohl an sich als auch für diesen Verwendungszweck wohlbekannt ist. Die Silanisierung erleichtert das Erreichen eines hohen Füllgrades im Komposit und wirkt sich günstig auf die mechanischen Eigenschaften des Komposits aus.

Ein erfindungsgemäßes Dentalglas-Kunststoff-Komposit besteht aus üblichen Dentalkunststoffen und einem erfindungsgemäßen Dentalglaspulver.

Vorzugsweise stimmt die Brechzahl n_{d} des Dentalglases mit der des Dentalkunststoffs besser als 0,05 überein, wobei die Brechzahl n_{d} des Dentalkunststoffs bevorzugt > 1,60 ist.

Zur Herstellung von als Zahnfüllung verwendbaren Dental-Kompositen wird das Glaspulver mit in der Zahnmedizin üblichen, härtbaren Kunstharzen gemischt. Als Kunstharze werden überwiegend UV-härtbare Harze auf Acrylat-, Methacrylat-, 2,2-Bis-[4-(3-Methacryloxy-2-hydroxypropoxy)-phenyl]-propan-(Bis-GMA-), Urethan-Methacrylat-, Alcandioldimethacrylat- oder Cyanacrylatbasis verwendet. Das zur Füllung verwendete Glaspulver liegt in den fertigen Kunstharzpasten in Gewichtsanteilen von bis zu 80 Gew.-% vor, wobei der Glaspulveranteil aus Festigkeitsgründen so hoch wie möglich zu wählen ist.

Tabelle 1 enthält 5 Ausführungsbeispiele und Eigenschaften (Brechzahl n_{d}, Aluminiumgleichwertdicke Al-GWD) im Zusammensetzungsbereich, in dem Dentalgläser mit niedrigen Brechzahlen zu finden sind, Tabelle 2 weitere 5 Beispiele mit hohen Brechzahlen.

**Tabelle 1 Beispiele für niedrigbrechende Gläser (Zusammensetzung in Gew. %)**

| Beispiel | 1.1 | 1.2 | 1.3 | 1.4 | 1.5 |
|---|---|---|---|---|---|
| | | | | | |
| SiO₂ | 38 | 20 | 25 | 23 | 30 |
| Al₂O₃ | 12 | 12 | 15 | 12 | 12 |
| B₂O₃ | 10 | 10 | 10 | 10 | 5 |
| Na₂O | 2 | 2 | 7 | 7 | 3 |
| SrO | - | 10 | - | 10 | - |
| ZnO | 6 | 15 | 15 | 6 | 15 |
| ZrO₂ | 3 | 3 | 6 | 3 | 6 |
| P₂O₅ | 5 | 10 | 5 | 10 | 10 |
| La₂O₃ | 4 | 2 | 2 | 4 | 4 |
| F | 20 | 16 | 15 | 15 | 15 |
| B₂O₃+ZnO+ZrO₂+ La₂O₃ | 23 | 30 | 33 | 23 | 30 |
| n_{d} | 1,514 | 1,569 | 1,530 | 1,535 | 1,565 |
| Al-GWD [mm] | 6,3 | 8,1 | 4,6 | 7,3 | 7,0 |

**Tabelle 2 Beispiele für hochbrechende Gläser (Zusammensetzung in Gew.-%)**

| Beispiel | 2.1 | 2.2 | 2.3 | 2.4 | 2.5 |
|---|---|---|---|---|---|
| | | | | | |
| SiO₂ | 30 | 25 | 30 | 22 | 20 |
| Al₂O₃ | 5 | 10 | 7 | 13 | 10 |
| B₂O₃ | 5 | 2 | 5 | 2 | 2 |
| Na₂O | 5 | 7 | 6 | 2 | 5 |
| K₂O | - | - | - | 2 | 5 |
| Cs₂O | - | 3 | - | 5 | 3 |
| Na₂O+K₂O+Cs₂O | 5 | 10 | 6 | 9 | 13 |
| CaO | 5 | - | - | - | 5 |
| SrO | 24 | 15 | 23 | 20 | 15 |
| ZnO | 10 | 15 | 10 | 15 | 14 |
| ZrO₂ | 8 | 8 | 4 | 7 | 9 |
| P₂O₅ | - | 4 | 4 | 3 | 2 |
| La₂O₃ | 3 | 6 | 8 | 6 | 8 |
| F | 5 | 5 | 3 | 3 | 2 |
| B₂O₃+ ZnO + ZrO₂ + La₂O₃ | 26 | 31 | 27 | 30 | 33 |
| n_{d} | 1,608 | 1,601 | 1,598 | 1,634 | 1,656 |
| Al-GWD [mm] | 11,4 | 11,3 | 11,3 | 12,3 | 11,9 |

**Tabelle 3 Schmelzbeispiel für 100 kg berechnetes Dentalglas (entsprechend Tabelle 1, Beispiel 1.4)**

| Komponente | Gew.- % | Rohstoff | Einwaage/kg |
|---|---|---|---|
| SiO₂ | 23,0 | SiO₂ | 23,01 |
| Al₂O₃ | 12,0 | Al(OH)₃ | 4,57 |
| Na₂O | 7,0 | Na₃AlF₆ | 15,81 |
| P₂O₅ | 10,0 | Al(PO₃)₃ | 12,53 |
| B₂O₃ | 10,0 | H₃BO₃ | 17,77 |
| SrO | 10,0 | SrF₂ | 12,50 |
| ZnO | 6,0 | ZnO | 6,01 |
| ZrO₂ | 3,0 | ZrO₂ | 3,06 |
| La₂O₃ | 4,0 | La₂O₃ | 4,01 |
| F | 15,0 | AlF₃ | 4,49 |
| Summe | 100,0 | | 103,76 |

## Patentansprüche

1. Bariumfreies röntgenopakes Dentalglas,
**gekennzeichnet durch**
eine Zusammensetzung (in Gew.-%) von:
| | |
|---|---|
| SiO₂ | 20 - 45 |
| Al₂O₃ | 5 - 35 |
| B₂O₃ | 0 - 10 |
| Na₂O | 1 - 10 |
| K₂O | 0 - 8 |
| Cs₂O | 0 - 8 |
| Na₂O+K₂O+Cs₂O | 1 - 15 |
| CaO | 0 - 8 |
| SrO | 0 - 27 |
| ZnO | 2 - 20 |
| ZrO₂ | 2 - 10 |
| P₂O₅ | 0 - 10 |
| La₂O₃ | 0 - 10 |
| F | 2 - 20 |
| B₂O₃ + ZnO + ZrO₂ + La₂O₃ | > 20, |
und eine Brechzahl n_{d} von 1,47 bis 1,70.

2. Dentalglas nach Anspruch 1,
**gekennzeichnet durch**
eine Zusammensetzung (in Gew.-%) von:
| | |
|---|---|
| SiO₂ | 20 - 45 |
| Al₂O₃ | 7 - 35 |
| B₂O₃ | 0,5 - 10 |
| Na₂O | 2 - 10 |
| K₂O | 0 - 8 |
| Cs₂O | 0 - 8 |
| Na₂O+K₂O+Cs₂O | 2 - 10 |
| CaO | 0 - 5 |
| SrO | 0 - 25 |
| ZnO | 2 - 15 |
| ZrO₂ | 2 - 6 |
| P₂O₅ | 2 - 10 |
| La₂O₃ | 0 - 5 |
| F | 7 - 20 |
| B₂O₃ + ZnO + ZrO₂ + La₂O₃ | > 20, |
und eine Brechzahl n_{d} von 1,47 bis 1,59.

3. Dentalglas nach Anspruch 1 oder 2,
**gekennzeichnet durch**
eine Zusammensetzung (in Gew.-%) von:
| | |
|---|---|
| SiO₂ | 20 - 44 |
| Al₂O₃ | 12 - 22 |
| B₂O₃ | 5 - 10 |
| Na₂O | 2 - 8 |
| CaO | 0 - 4 |
| SrO | 0 - 18,5 |
| ZnO | 3 - 15 |
| ZrO₂ | 3 - 6 |
| P₂O₅ | 4 - 10 |
| La₂O₃ | 0 - 4 |
| F | 10 - 20 |
| B₂O₃ + ZnO + ZrO₂ + La₂O₃ | > 20, |
und eine Brechzahl n_{d} von 1,49 bis 1,57.

4. Dentalglas nach Anspruch 1,
**gekennzeichnet durch**
eine Zusammensetzung (in Gew.-%) von:
| | |
|---|---|
| SiO₂ | 20 - 30 |
| Al₂O₃ | 5 - 25 |
| B₂O₃ | 0 - 10 |
| Na₂O | 3 - 10 |
| K₂O | 0 - 8 |
| Cs₂O | 0 - 8 |
| Na₂O+K₂O+Cs₂O | 3 - 15 |
| CaO | 0 - 8 |
| SrO | 0 - 25 |
| ZnO | 2 - 20 |
| ZrO₂ | 2 - 10 |
| P₂O₅ | 0 - 10 |
| La₂O₃ | 0 - 10 |
| F | 2 - 10 |
| B₂O₃ + ZnO + ZrO₂ + La₂O₃ | > 20, |
und eine Brechzahl n_{d} von 1,59 bis 1,70.

5. Dentalglas nach Anspruch 1 oder 4
**gekennzeichnet durch**
eine Zusammensetzung (in Gew.-%) von:
| | |
|---|---|
| SiO₂ | 20 - 30 |
| Al₂O₃ | 5 - 25 |
| B₂O₃ | 1 - 10 |
| Na₂O | 3 - 10 |
| K₂O | 0 - 8 |
| Cs₂O | 0 - 8 |
| Na₂O+K₂O+Cs₂O | 5 - 15 |
| CaO | 0 - 5 |
| SrO | 10 - 25 |
| ZnO | 8 - 20 |
| ZrO₂ | 4 - 10 |
| P₂O₅ | 2 - 10 |
| La₂O₃ | 3 - 10 |
| F | 2 - 7 |
| B₂O₃ + ZnO + ZrO₂ + La₂O₃ | > 20, |
und eine Brechzahl von 1,59 bis 1,67.

6. Dentalglas nach Anspruch1,4 oder 5
**gekennzeichnet durch**
eine Zusammensetzung (in Gew.-%) von:
| | |
|---|---|
| SiO₂ | 20 - 30 |
| Al₂O₃ | 5 - 15 |
| B₂O₃ | 2 - 5 |
| Na₂O | 3 - 7 |
| K₂O | 0 - 5 |
| Cs₂O | 0 - 5 |
| Na₂O + K₂O + Cs₂O | 5 - 13 |
| CaO | 0 - 5 |
| SrO | 15 - 24 |
| ZnO | 10 - 15 |
| ZrO₂ | 4 - 9 |
| P₂O₅ | 2 - 5 |
| La₂O₃ | 3 - 8 |
| F | 2 - 5 |
| B₂O₃ + ZnO + ZrO₂ + La₂O₃ | > 20, |
und eine Brechzahl von 1,59 bis 1,66.

7. Dentalglas nach wenigstens einem der Ansprüche 1 bis 6,
**gekennzeichnet durch**
einen zusätzlichen Gehalt von bis zu 10 Gew.-% eines oder mehrerer Oxide der Gruppe Sc₂O₃, Y₂O₃, Nb₂O₅, Gd₂O₃, Yb₂O₃, wobei die Summe dieser Oxide nicht größer als 10 Gew.-% ist.

8. Dentalglas nach wenigstens einem der Ansprüche 1 bis 7,
**gekennzeichnet durch**
einen zusätzlichen Gehalt von bis zu 10 Gew.-% eines oder mehrerer Oxide der Gruppe HfO₂, Ta₂O₅, WO₃, wobei die Summe dieser Oxide nicht größer als 10 Gew.-% ist.

9. Dentalglas nach wenigstens einem der Ansprüche 1 bis 8,
**gekennzeichnet durch**
eine Aluminiumgleichwertdicke von mindestens 4 mm.

10. Dentalglas nach wenigstens einem der Ansprüche 1 bis 9,
**dadurch gekennzeichnet,**
**daß** das Dentalglaspulver eine mittlere Teilchengröße von ≤ 10 µm, insbesondere von 0,5 bis 5 µm besitzt.

11. Dentalglas-Kunststoff-Komposit, enthaltend einen Dentalkunststoff und ein Dentalglas nach wenigstens einem der Ansprüche 1 bis 10.

12. Dentalglas-Kunststoff-Komposit nach Anspruch 11,
**dadurch gekennzeichnet,**
**daß** der Dentalkunststoff ein überwiegend UV-härtbares Harz auf Acrylat-, Methacrylat-, 2,2-Bis-[4-(3-Methacryloxy-2-hydroxypropoxy)-phenyl]-propan-(Bis-GMA-), Urethan-Methacrylat-, Alcandioldimethacrylat- oder Cyanacrylatbasis ist.

13. Dentalglas-Kunststoff-Komposit nach Anspruch 11 oder 12,
**dadurch gekennzeichnet,**
**daß** die Brechzahl n_{d} des Dentalglases mit der des Dentalkunststoffs besser als 0,05 übereinstimmt.

14. Dentalglas-Kunststoff-Komposit nach Anspruch 13,
**dadurch gekennzeichnet,**
**daß** die Brechzahl n_{d} des Dentalkunststoffs > 1,60 ist.

15. Dentalglas-Kunststoff-Komposit nach wenigstens einem der Ansprüche 11 bis 14,
**gekennzeichnet durch**
einen Dentalglasgehalt von bis zu 80 Gew.-%.

## Claims

1. Barium-free, X-ray-opaque dental glass, **characterized by** a composition (in % by weight) of:
| | |
|---|---|
| SiO₂ | 20 - 45 |
| Al₂O₃ | 5 - 3 5 |
| B₂O₃ | 0 - 10 |
| Na₂O | 1 - 10 |
| K₂O | 0 - 8 |
| Cs₂O | 0 - 8 |
| Na₂O + K₂O + Cs₂O | 1 - 15 |
| CaO | 0 - 8 |
| SrO | 0 - 27 |
| ZnO | 2 - 20 |
| ZrO₂ | 2 - 10 |
| P₂O₅ | 0 - 10 |
| La₂O₃ | 0 - 10 |
| F | 2 - 20 |
| B₂O₃ + ZnO + ZrO₂ + La₂O₃ | > 20 |
and a refractive index n_{d} of from 1.47 to 1.70.

2. Dental glass according to Claim 1, **characterized by** a composition (in % by weight) of:
| | |
|---|---|
| SiO₂ | 20 - 45 |
| Al₂O₃ | 7 - 35 |
| B₂O₃ | 0.5 - 10 |
| Na₂O | 2 - 10 |
| K₂O | 0 - 8 |
| Cs₂O | 0 - 8 |
| Na₂O + K₂O + Cs₂O | 2 - 10 |
| CaO | 0 - 5 |
| SrO | 0 - 25 |
| ZnO | 2 - 15 |
| ZrO₂ | 2 - 6 |
| P₂O₅ | 2 - 10 |
| La₂O₃ F | 0 - 5 7 - 20 |
| B₂O₃ + ZnO + ZrO₂ + La₂O₃ | > 20 |
and a refractive index n_{d} of from 1.47 to 1.59.

3. Dental glass according to Claim 1 or 2, **characterized by** a composition (in % by weight) of:
| | |
|---|---|
| SiO₂ | 20 - 44 |
| Al₂O₃ | 12 - 22 |
| B₂O₃ | 5 - 10 |
| Na₂O | 2 - 8 |
| CaO | 0 - 4 |
| SrO | 0 - 18.5 |
| ZnO | 3 - 15 |
| ZrO₂ | 3 - 6 |
| P₂O₅ | 4 - 10 |
| La₂O₃ | 0 - 4 |
| F | 10 - 20 |
| B₂O₃ + ZnO + ZrO₂ + La₂O₃ | > 20 |
and a refractive index n_{d} of from 1.49 to 1.57.

4. Dental glass according to Claim 1, **characterized by** a composition (in % by weight) of:
| | |
|---|---|
| SiO₂ | 20 - 30 |
| Al₂O₃ | 5 - 25 |
| B₂O₃ | 0 - 10 |
| Na₂O | 3 - 10 |
| K₂O | 0 - 8 |
| Cs₂O | 0 - 8 |
| Na₂O + K₂O + Cs₂O | 3 - 15 |
| CaO | 0 - 8 |
| SrO | 0 - 25 |
| ZnO | 2 - 20 |
| ZrO₂ | 2 - 10 |
| P₂O₅ | 0 - 10 |
| La₂O₃ | 0 - 10 |
| F | 2 - 10 |
| B₂O₃ + ZnO + ZrO₂ + La₂O₃ | > 20 |
and a refractive index n_{d} of from 1.59 to 1.70.

5. Dental glass according to Claim 1 or 4, **characterized by** a composition (in % by weight) of:
| | |
|---|---|
| SiO₂ | 20 - 30 |
| Al₂O₃ | 5 - 25 |
| B₂O₃ | 1 - 10 |
| Na₂O | 3 - 10 |
| K₂O | 0 - 8 |
| Cs₂O | 0 - 8 |
| Na₂O + K₂O + Cs₂O | 5 - 15 |
| CaO | 0 - 5 |
| SrO | 10 - 25 |
| ZnO | 8 - 20 |
| ZrO₂ | 4 - 10 |
| P₂O₅ | 2 - 10 |
| La₂O₃ | 3 - 10 |
| F | 2 - 7 |
| B₂O₃ + ZnO + ZrO₂ + La₂O₃ | > 20 |
and a refractive index of from 1.59 to 1.67.

6. Dental glass according to Claim 1, 4 or 5, **characterized by** a composition (in % by weight) of:
| | |
|---|---|
| SiO₂ | 20 - 30 |
| Al₂O₃ | 5 - 15 |
| B₂O₃ | 2 - 5 |
| Na₂O | 3 - 7 |
| K₂O | 0 - 5 |
| Cs₂O | 0 - 5 |
| Na₂O + K₂O + Cs₂O | 5 - 13 |
| CaO | 0 - 5 |
| SrO | 15 - 24 |
| ZnO | 10 - 15 |
| ZrO₂ | 4 - 9 |
| P₂O₅ | 2 - 5 |
| La₂O₃ | 3 - 8 |
| F | 2 - 5 |
| B₂O₃ + ZnO + ZrO₂ + La₂O₃ | > 20 |
and a refractive index of from 1.59 to 1.66.

7. Dental glass according to at least one of Claims 1 to 6, **characterized by** an additional content of up to 10% by weight of one or more oxides from the group consisting of Sc₂O₃, Y₂O₃, Nb₂O₅, Gd₂O₃ and Yb₂O₃, where the sum of these oxides is not greater than 10% by weight.

8. Dental glass according to at least one of Claims 1 to 7, **characterized by** an additional content of up to 10% by weight of one or more oxides from the group consisting of HfO₂, Ta₂O₅ and WO₃, where the sum of these oxides is not greater than 10% by weight.

9. Dental glass according to at least one of Claims 1 to 8, **characterized by** an aluminium equivalent thickness of at least 4 mm.

10. Dental glass according to at least one of Claims 1 to 9, **characterized in that** the dental glass powder has a mean particle size of ≤ 10 µm, in particular from 0.5 to 5 µm.

11. Dental glass/polymer composite comprising a dental polymer and a dental glass according to at least one of Claims 1 to 10.

12. Dental glass/polymer composite according to Claim 11, **characterized in that** the dental polymer is a predominantly UV-curable resin based on acrylate, methacrylate, 2,2-bis[4-(3-methacryloxy-2-hydroxypropoxy)phenyl]propane (bis-GMA), urethane methacrylate, alkanediol dimethacrylate or cyanoacrylate.

13. Dental glass/polymer composite according to Claim 11 or 12, **characterized in that** the refractive index n_{d} of the dental glass corresponds to that of the dental polymer within 0.05.

14. Dental glass/polymer composite according to Claim 13, **characterized in that** the refractive index n_{d} of the dental polymer is > 1.60.

15. Dental glass/polymer composite according to at least one of Claims 11 to 14, **characterized by** a dental glass content of up to 80% by weight.

## Revendications

1. Verre dentaire radio-opaque sans baryum, **caractérisé par** une composition (en % en poids) de :
| | |
|---|---|
| SiO₂ | 20 - 45 |
| Al₂O₃ | 5 - 35 |
| B₂O₃ | 0 - 10 |
| Na₂O | 1 - 10 |
| K₂O | 0 - 8 |
| Cs₂O | 0 - 8 |
| Na₂O + K₂O + Cs₂O | 1 - 15 |
| CaO | 0 - 8 |
| SrO | 0 - 27 |
| ZnO | 2 - 20 |
| ZrO₂ | 2 - 10 |
| P₂O₅ | 0 - 10 |
| La₂O₃ | 0 - 10 |
| F | 2 - 20 |
| B₂O₃ + ZnO + ZrO₂ + La₂O₃ | > 20 |
et un indice de réfraction n_{d} de 1,47 à 1,70.

2. Verre dentaire selon la revendication 1, **caractérisé par** une composition (en % en poids) de :
| | |
|---|---|
| SiO₂ | 20 - 45 |
| Al₂O₃ | 7 - 35 |
| B₂O₃ | 0,5 - 10 |
| Na₂O | 2 - 10 |
| K₂O | 0 - 8 |
| Cs₂O | 0 - 8 |
| Na₂O + K₂O + Cs₂O | 2 - 10 |
| CaO | 0 - 5 |
| SrO | 0 - 25 |
| ZnO | 2 - 15 |
| ZrO₂ | 2 - 6 |
| P₂O₅ | 2 - 10 |
| La₂O₃ | 0 - 5 |
| F | 7 - 20 |
| B₂O₃ + ZnO + ZrO₂ + La₂O₃ | > 20 |
et un indice de réfraction n_{d} de 1,47 à 1,59.

3. Verre dentaire selon la revendication 1 ou 2, **caractérisé par** une composition (en % en poids) de :
| | |
|---|---|
| SiO₂ | 20 - 44 |
| Al₂O₃ | 12 - 22 |
| B₂O₃ | 5 - 10 |
| Na₂O | 2 - 8 |
| CaO | 0 - 4 |
| SrO | 0 - 18,5 |
| ZnO | 3 - 15 |
| ZrO₂ | 3 - 6 |
| P₂O₅ | 4 - 10 |
| La₂O₃ | 0 - 4 |
| F | 10 - 20 |
| B₂O₃ + ZnO + ZrO₂ + La₂O₃ | > 20 |
et un indice de réfraction n_{d} de 1,49 à 1,57.

4. Verre dentaire selon la revendication 1, **caractérisé par** une composition, en % en poids de :
| | |
|---|---|
| SiO₂ | 20 - 30 |
| Al₂O₃ | 5 - 25 |
| B₂O₃ | 0 - 10 |
| Na₂O | 3 - 10 |
| K₂O | 0 - 8 |
| Cs₂O | 0 - 8 |
| Na₂O + K₂O + Cs₂O | 3 - 15 |
| CaO | 0 - 8 |
| SrO | 0 - 25 |
| ZnO | 2 - 20 |
| ZrO₂ | 2 - 10 |
| P₂O₅ | 0 - 10 |
| La₂O₃ | 0 - 10 |
| F | 2 - 10 |
| B₂O₃ + ZnO + ZrO₂ + La₂O₃ | > 20 |
et un indice de réfraction n_{d} de 1,59 à 1,70.

5. Verre dentaire selon la revendication 1 ou 4, **caractérisé par** une composition (en % en poids) de :
| | |
|---|---|
| SiO₂ | 20 - 30 |
| Al₂O₃ | 5 - 25 |
| B₂O₃ | 1 - 10 |
| Na₂O | 3 - 10 |
| K₂O | 0 - 8 |
| Cs₂O | 0 - 8 |
| Na₂O + K₂O + Cs₂O | 5 - 15 |
| CaO | 0 - 5 |
| SrO | 10 - 25 |
| ZnO | 8 - 20 |
| ZrO₂ | 4 - 10 |
| P₂O₅ | 2 - 10 |
| La₂O₃ | 3 - 10 |
| F | 2 - 7 |
| B₂O₃ + ZnO + ZrO₂ + La₂O₃ | > 20 |
et un indice de réfraction de 1,59 à 1,67.

6. Verre dentaire selon la revendication 1, 4 ou 5, **caractérisé par** une composition (en % en poids) de :
| | |
|---|---|
| SiO₂ | 20 - 30 |
| Al₂O₃ | 5 - 15 |
| B₂O₃ | 2 - 5 |
| Na₂O | 3 - 7 |
| K₂O | 0 - 5 |
| Cs₂O | 0 - 5 |
| Na₂O + K₂O + Cs₂O | 5 - 13 |
| CaO | 0 - 5 |
| SrO | 15 - 24 |
| ZnO | 10 - 15 |
| ZrO₂ | 4 - 9 |
| P₂O₅ | 2 - 5 |
| La₂O₃ | 3 - 8 |
| F | 2 - 5 |
| B₂O₃ + ZnO + ZrO₂ + La₂O₃ | > 20 |
et un indice de réfraction de 1,59 à 1,66.

7. Verre dentaire selon au moins l'une des revendications 1 à 6, **caractérisé en ce qu'**il contient en outre jusqu'à 10 % en poids d'un ou plusieurs oxydes du groupe Sc₂O₃, Y₂O₃, Nb₂O₅, Gd₂O₃, Yb₂O₃, la somme de ces oxydes n'étant pas supérieure à 10 % en poids.

8. Verre dentaire selon au moins l'une des revendications 1 à 7, **caractérisé en ce qu'**il contient en outre jusqu'à 10 % en poids d'un ou plusieurs oxydes du groupe HfO₂, Ta₂O₅, WO₃, la somme de ces oxydes n'étant pas supérieure à 10 % en poids.

9. Verre dentaire selon au moins l'une des revendications 1 à 8, **caractérisé par** une épaisseur équivalente d'aluminium d'au moins 4 mm.

10. Verre dentaire selon au moins l'une des revendications 1 à 9, **caractérisé en ce que** la poudre de verre dentaire présente une granulométrie moyenne ≤ 10 µm, en particulier de 0,5 à 5 µm.

11. Composite verre dentaire-plastique dentaire, contenant un plastique dentaire et un verre dentaire selon au moins l'une des revendications 1 à 10.

12. Composite verre dentaire-plastique dentaire selon la revendication 11, **caractérisé en ce que** le plastique dentaire est une résine essentiellement durcissable aux UV, à base d'acrylate, de méthacrylate, de 2,2-bis-[4-(3-méthacryloxy-2-hydroxypropoxy)-phényl]-propane (Bis-GMA-), de méthacrylate d'uréthanne, de diméthacrylate d'alcanediol ou de cyanoacrylate.

13. Composite verre dentaire-plastique dentaire selon la revendication 11 ou 12, **caractérisé en ce que** l'indice de réfraction nd du verre dentaire coïncide avec celui du plastique dentaire, avec un écart inférieur à 0,05.

14. Composite verre dentaire-plastique dentaire selon la revendication 13, **caractérisé en ce que** l'indice de réfraction nd du plastique dentaire est > 1,60.

15. Composite verre dentaire-plastique dentaire selon au moins l'une des revendications 11 à 14, **caractérisé par** une teneur en verre dentaire allant jusqu'à 80 % en poids.
